(19) Europäisches Patentamt
European Patent Office
Office européen des brevets

(11) EP 1 792 598 A1

(12) EUROPEAN PATENT APPLICATION
published in accordance with Art. 158(3) EPC

(43) Date of publication:
06.06.2007 Bulletin 2007/23

(51) Int Cl.:
A61K 6/00 (2006.01)

(21) Application number: 05783324.6

(86) International application number:
PCT/JP2005/017148

(22) Date of filing: 16.09.2005

(87) International publication number:
WO 2006/033301 (30.03.2006 Gazette 2006/13)

(84) Designated Contracting States:
DE ES FR GB IT

(30) Priority: 21.09.2004 JP 2004273674

(71) Applicant: Shiseido Company, Limited
Chuo-ku
Tokyo 104-8010 (JP)

(72) Inventors:
• TAKADA, Hirotaka, c/o SHISEIDO RESEARCH CENTER
Yokohama-shi, Kanagawa 224-8558 (JP)
• OHNO, Kazuhisa, c/o SHISEIDO RESEARCH CENTER
Yokohama-shi, Kanagawa 224-8558 (JP)
• SUDA, Yukimitsu, c/o SHISEIDO RESEARCH CENTER
Yokohama-shi, Kanagawa 224-8558 (JP)

(74) Representative: Merkle, Gebhard
TER MEER STEINMEISTER & PARTNER GbR,
Patentanwälte,
Mauerkircherstrasse 45
81679 München (DE)

(54) SPECULAR-GLOSS NAIL ENAMELS

(57) The present invention provides (a) a nail enamel characteristically containing noble metal colloidal particles having an average particle size of 10-100 nm whose concentration is 5-50 wt% of the total amount of the nail enamel. It also provides a 2-formula type nail enamel that is a combination of this nail enamel (formula 2) and a base coat (formula 1) containing (b) a film forming agent and (c) water and/or an organic solvent.

The object of the present invention is to provide a nail enamel that is suitable for actual use and after application gives a finish having a mirror gloss.

EP 1 792 598 A1

## EP 1 792 598 A1

**Description**

TECHNICAL FIELD

The present invention relates to a mirror gloss nail enamel. More specifically, it relates to a nail enamel that is highly durable and gives a mirror gloss finish after application.

BACKGROUND ART

Nail enamels' functions include beautifying the appearance, maintaining the health of the nails and protecting the nails from physical impacts and chemical substances.

Of these, beautiful appearance is one of the most important items; in recent years there are increased needs for novelty and variations in the texture of the color tone of the appearance; and as a result various color materials and powders in addition to conventionally used pigments are blended into nail enamels.

Raw materials of such color materials and powders range widely; lustrous powders such as titanated mica, bismuth oxychloride, fish scale flakes, and aluminum powder as well as metal deposited resin film, embossed metal deposited film, laminated resin films, glass flakes coated with metal or metal oxide, thin flake aluminum powder, etc. have been developed. In many cases several kinds of these are used together.

Also, for blending metals in cosmetics, there is technology to blend in lustrous powder derived from the aforementioned metals or metal oxides; on the other hand there is technology to utilize the plasmon absorption of fine particles of noble metals such as gold and silver to use them as coloring agents in cosmetics.

Methods for this technology include a method as represented by Patent Document 1 in which hydrosols of noble metal fine particles are prepared and then directly blended into a cosmetic and a method as disclosed in Patent Document 3 in which gold fine particles are fixed on a material that serves as a carrier before being blended in a cosmetic.

[Patent Document 1] Japanese Patent Laid-Open H4-89416 bulletin
[Patent Document 2] Japanese Patent Laid-Open H4-173724 bulletin
[Patent Document 3] Japanese Patent Laid-Open H1-215865 bulletin

DISCLOSURE OF INVENTION

[Problem that the present invention aims to solve]

As described above, there are many existing technologies for color tones and textures of nail enamels and cosmetics; there are various types of nail enamel products on the market.

However, there is no nail enamel that gives a mirror glossy finish; a nail enamel containing the aforementioned powders has luster but its finish is not mirror-like and a completely different technology is needed for such a finish. When noble metal colloidal fine particles are simply blended in a nail enamel, gloss may result but the water resistance and wear resistance are not enough for practical use.

The present invention was carried out in view of the problems of the aforementioned conventional technology; its object is to provide a new nail enamel having high durability and a mirror gloss finish.

[Means to solve the Problem]

That is, the present invention provides (a) a nail enamel characteristically containing noble metal colloidal particles having an average particle size of 10-100 nm whose concentration is 5-50 wt% of the total amount of the nail enamel.

Also, the present invention provides the aforementioned nail enamel wherein said noble metal colloidal particles are obtained by using a manufacturing method including a manufacturing process in which a metal compound is reduced in the presence of a polymer pigment dispersant to obtain a noble metal colloidal particle solution and a concentration process in which the noble metal colloidal particle solution obtained in said manufacturing process is ultrafiltrated or the solvent is evaporated.

Furthermore, the present invention provides a 2-formula type nail enamel comprising the following formula A and formula B.

Formula A: The nail enamel of claim 1 or 2
Formula B: A base coat containing (b) a film forming agent and (c) water and/or an organic solvent

Also, the present invention provides the aforementioned 2-formula type nail enamel wherein said formula A additionally contains (d) ethanol.

Furthermore, the present invention provides the aforementioned 2-formula type nail enamel wherein said formula B contains (e) a plasticizer having an I0B value of 0.3-1.0 and a molecular weight of 500 or less.

Also, the present invention provides the aforementioned 2-formula type nail enamel wherein (b) the film forming agent is a combination of (b1) nitrocellulose and (b2) one, two or more chosen from a group consisting of sucrose benzoate, trimellitic acid type alkyd resin, and toluenesulfon amide resin.

Also, the present invention provides the aforementioned nail enamel wherein said formula A's noble metal is silver.

Furthermore, the present invention provides a manicure method having a step in which said formula B is applied on the nails, followed by a step in which said formula A is applied on the nails.

[Effects of the invention]

(1) According to the present invention, a nail enamel having a mirror gloss of a noble metal with a gorgeous luster can be provided.

(2) The nail enamel of the present invention particularly improves water resistance and wear resistance by using formula B as a base coat and applying the nail enamel on this base coat.

Combined use of this base coat also improves the mirror gloss and provides a mirror gloss that has less change in the color tone over time. When the base coat is used, the metal gloss is optimally manifested when the diffusion medium of formula A is water or ethanol; if it is butyl acetate, then the metal gloss is not manifested. Therefore, formula A should preferably use an ethanol solvent that does not contain butyl acetate. When the diffusion medium of formula A is water (when formula A is a water system having water as the main ingredient), the water resistance and wear resistance will be inferior.

(3) When formula B of the nail enamel of the present invention is used not as the base coat but as the top coat, and if formula B contains an organic solvent, the mirror film may re-dissolve and the mirror gloss may disappear. When formula B doesn't contain an organic solvent, a mirror gloss with less color tone change over time can be obtained; however, the water resistance and the wear resistance are inferior.

On the other hand, if formula B is additionally used as both the base coat and the top coat, the result is not preferable in terms of the mirror gloss and the color tone change over time.

BEST MODE FOR CARRYING OUT THE INVENTION

The nail enamel of the present invention is a nail enamel that has high durability, a mirror gloss, and an image-reflecting effect.

Selection of the noble metal used in the present invention is not limited in particular; examples include gold, silver, ruthenium, rhodium, palladium, osmium, iridium and platinum. Among them, gold, silver, platinum, and palladium are particularly preferable; silver is even more preferable because of the color tone and price.

The present invention is a nail enamel characteristically containing noble metal colloidal particles having an average particle size of 10-100 nm whose concentration is 5-50 wt% of the total amount of the nail enamel. After the solvent evaporates and the nail enamel becomes a film formed on the nails, the metal particle content in the nail enamel (not including the base coat) is preferably 90 wt% or higher, and more preferably 93 wt% or higher. If it is less than 90 wt%, then the formed film cannot have a beautiful mirror gloss.

The noble metal used is preferably blended in the form of fine colloidal particles. Even more preferable is to blend it in the form of a noble metal colloid dispersed in a dispersing medium. This makes it easier to apply the nail enamel with a brush and such. The dispersing medium is preferably volatile at ordinary temperatures since the film needs to be formed at ordinary temperatures. Specifically, alcohols such as ethanol, organic solvents such as ethyl acetate, butyl acetate, and toluene, and water can be used; the nail enamel should preferably use an ethanol-type solvent having ethanol as the main ingredient. This ethanol-type solvent preferably should not contain other organic solvents such as ethyl acetate, butyl acetate, and toluene.

The aforementioned noble metal colloidal particles are preferably obtained by using a manufacturing method including a manufacturing process in which a metal compound is reduced in the presence of a polymer pigment dispersant to obtain a noble metal colloidal particle solution and a concentration process in which the noble metal colloidal particle solution obtained in said manufacturing process is ultrafiltrated or the solvent is evaporated.

For example, the noble metal colloidal particle solution described in Japanese Patent Laid-Open 2003-103158 bulletin can be preferably used. Also, commercially available products such as the Finesphere series from Nippon Paint Co.,

Ltd. can be used.

The average particle size of the aforementioned noble metal colloid is 10-100 nm. If it is less than 10 nm, then the metallic gloss coating film may be hard to form. If it is over 100 nm, then the smoothness of the formed coating film becomes poor and the mirror gloss is harder to obtain; also, the storage stability in the dispersing medium may decrease. Furthermore, if it is less than 10 nm, then the reflectance decreases, which may make it harder to obtain the perceived mirror gloss.

The aforementioned noble metal colloidal solution is preferably dispersed and stabilized by means of a high molecular weight pigment dispersant. The following can be preferably used for the high molecular weight pigment dispersant of the present invention. That is:

(1) A comb-structured polymer having a pigment affinitive group in the main chain and/or multiple side chains and also having multiple side chains that constitute the solvation portion.
(2) A polymer having in its main chain multiple pigment affinitive portions composed of pigment affinitive groups.
(3) A straight chain polymer having at one end of its main chain a pigment affinitive portion composed of a pigment affinitive group.
Here, the aforementioned pigment affinitive group refers to a functional group that adheres strongly to the pigment surface; examples for organosols include a tertiary amino group, quaternary ammonium group, heterocyclic group, hydroxyls group, and carboxyl group, and examples for hydrosols include a phenyl group, lauryl group, stearyl group, dodecyl group, and oleyl group. In the present invention, the aforementioned pigment affinitive group manifests strong affinity to noble metals. The aforementioned high molecular weight pigment dispersant, by virtue of having the aforementioned pigment affinitive group, can manifest sufficient performance as a protective colloid for noble metals.

The aforementioned comb-structured polymer (1) has a structure in which multiple side chains having the aforementioned pigment affinitive group as well as multiple side chains constituting the solvation portion bind to the main chain as if they are comb teeth. In this specification, the aforementioned structure is called the "comb structure." In the aforementioned comb-structured polymer (1), a plurality of the aforementioned pigment affinitive groups can exist in the middle of the side chains or in the main chain in addition to at the ends of the side chains. The aforementioned solvation portion refers to a portion having affinity to the solvent; its structure is hydrophilic or hydrophobic. The solvation portion is, for example, composed of water soluble polymerized chains, lipophilic polymerized chains, etc.

Selection of the aforementioned comb-structured polymer (1) is not limited in particular; examples include a poly (ethyleneimine) or acid salt thereof disclosed in Japanese Patent Laid-Open H5-177123 bulletin that has a structure having one or more poly (carbonyl-C3-C6-alkyleneoxy) chains wherein each of such chains has 3-80 carbonyl-C3-C6-alkylenoxy groups and is bonded to poly (ethyleneimine) via amide or salt cross-linking groups, a structure disclosed in Japanese Patent Laid-Open S54-37082 bulletin that is a reaction product of poly (lower alkylene) imine and polyester having free carboxylic acid groups wherein at least two polyester sequences are bonded to each poly (lower alkylene) imine sequence, and a pigment dispersant disclosed in Japanese Patent Publication H7-24746 bulletin obtained by reacting a high molecular weight epoxy compound with an amine compound and a pre-polymer containing carboxyl groups having a number average molecular weight of 300-7,000, simultaneously or in any order.

Commercially available products can also be used for the aforementioned high molecular weight pigment dispersant. Examples of the aforementioned commercially available products include Solsperse 20000, Solsperse 24000, Solsperse 26000, Solsperse 27000, Solsperse 28000, and Solsperse 41090 (from Avecia Ltd.), Disperbyk 160, Disperbyk 161, Disperbyk 162, Disperbyk 163, Disperbyk 166, Disperbyk 170, Disperbyk 180, Disperbyk 181, Disperbyk 182, Disperbyk 183, Disperbyk 184, Disperbyk 190, Disperbyk 191, Disperbyk 192, Disperbyk 2000, and Disperbyk 2001 (from BYK Chemie), Polymer 100, Polymer 120, Polymer 150, Polymer 400, Polymer 401, Polymer 402, Polymer 403, Polymer 450, Polymer 451, Polymer 452, Polymer 453, EFKA-46, EFKA-47, EFKA-48, EFKA-49, EFKA-1501, EFKA-1502, EFKA-4540, and EFKA-4550 (from EFKA Chemical), Flowlen DOPA-158, Flowlen DOPA-22, Flowlen DOPA-17, Flowlen G-700, Flowlen TG-720W, Flowlen 730W, Flowlen 740W, and Flowlen 745W (from Kyoeisha Chemical 1 Co., Ltd.), Ajisper PA111, Ajisper PB711, Ajisper PB811, Ajisper PB821, and Ajisper PW911 (from Ajinomoto), Johncryl 678, Johncryl 679, and Johncryl 62 (from Johnson Polymer). These can be used either independently or in combinations of two or more.

The aforementioned high molecular weight pigment dispersant is either a graft structure having the pigment affinitive groups in side chains as well as the side chains that constitute the solvation portion [the aforementioned comb-structured polymer (1)] or a structure whose main chain has the pigment affinitive groups [the aforementioned copolymer (2) and the aforementioned straight chain polymer (3)], and therefore dispersion of the colloidal particles is satisfactory, which makes it a preferable protective colloid for the noble metal colloidal particles. By using the aforementioned high molecular weight pigment dispersant, a noble metal colloidal particle dispersion containing a high concentration of noble metal colloidal particles can be stabilized.

For the nail enamel of the present invention, the mirror gloss of the finish improves visually and the durability of the perceived mirror gloss film increases when the base coat (formula B), which is a film forming agent dissolved or dispersed in water, alcohol, or a solvent, is first applied on the nails as formula 1 and then the nail enamel of the present invention (formula A) is applied on top as formula 2.

When the nail enamel of the present invention (formula A) is used as formula 2, the dispersion medium for it is preferably water or alcohol. Ethanol is particularly preferable. When ethanol is used, the ethanol content in formula A is preferably 20-95 wt%. If it is less than 20 wt%, then the wear resistance is inferior, and if it is over 95 wt%, then the noble metal content decreases and therefore the mirror gloss cannot be obtained: neither case is preferable.

Formula B, the base coat, is a base coat containing (b) a film forming agent and (c) water and/or an organic solvent. Conventional solvent type base coats and water type base coats mainly using water for the dispersion medium can be used.

For the (b) film forming agent of the solvent type base coat, cellulose type film agents such as ethyl cellulose, nitro cellulose, and cellulose acetate lactate, as well as acrylic resin, sulfonamide resin, sucrose benzoate, sucrose acetate lactate, phthalic acid type alkyd resin, trimellitic acid type alkyd resin can be used. Particularly preferable is a combination of (b1) nitro cellulose and (b2) one, two or more chosen from a group consisting of sucrose benzoate, trimellitic acid type alkyd resin, and toluenesulfonamide resin.

Examples of the (b) film forming agent for the water type base coat include acrylic resin, acrylic polymer emulsion, vinyl acetate emulsion, and polyvinyl alcohol.

The blend ratio of these film forming agents (b) are usually about 5-40 wt% of the total amount of the base coat.

It is more preferable that the base coat of the nail enamel of the present invention contain the plasticizer of film forming agent (b). The addition of the plasticizer can improve the wear resistance and water resistance of the mirror gloss nail enamel film applied on the base coat. For the plasticizer, camphor, citric esters, phthalic esters, sebacic esters, adipic esters, etc. can be used. The blend ratio is 5-60 wt%, preferably 10-50 wt%, of film forming agent (b) blended into the base coat. If it is less than 5 wt%, then sufficient wear resistance cannot be obtained; and if it is more than 60 wt%, then the base coat film becomes soft and the wear resistance decreases rather than increasing.

Preferable for the aforementioned plasticizer is (e) a plasticizer having an IOB value of 0.3-1.0 and a molecular weight of 500 or less.

When a water type polymer emulsion is used for film forming agent (b), the IOB value is preferably between 0.4 and 0.9. If it is 0.4 or less, then the compatibility with the film decreases and a homogeneous film may not be obtained; and if it is 0.9 or more, then the stability of the polymer emulsion may deteriorate over time.

The IOB (inorganic organic balance) value in the present invention is the inorganicity/organicity ratio calculated according to Fujita's calculation method disclosed in "Kagaku no Ryoiki (Chemistry and Related Fields)" Vol. 11, No. 10, 719-725 (1957), that is, a value represented by the following formula.

$$IOB\ value\ =\ \Sigma\ Inorganicity\ /\ \Sigma\ Organicity$$

For example, acetyl triethyl citrate, the most preferable plasticizer in the present invention, has an I0B of 0.86 and a molecular weight of 346.

The durability of the mirror gloss film of the nail enamel of the present invention can be further improved by additionally applying a top coat. In order to maintain a more beautiful mirror gloss, the solvent of the top coat should preferably be water. Considering the ease of application and the durability of the top coat, the film agent used should preferably be a polymer emulsion.

In addition to the aforementioned ingredients, other ingredients generally used in nail enamels can be blended as necessary into the nail enamel of the present invention as long as the effect of the present invention is not adversely affected.

Examples include alkyd resin, sulfonamide resin, sucrose benzoate, sucrose acetate butyrate, cellulose derivatives, acrylic acid alkyl copolymer solution, vinyl acetate emulsion, acrylic polymer emulsion, polyvinyl alcohol, plasticizers, perfume, dyes, drugs, humectants, ultraviolet absorbents, fillers, surfactants, and metal soaps.

EXAMPLES

The present invention is described in detail below by referring to Examples. The present invention is not limited to these examples. The blend ratios are in mass-percentage units unless specified otherwise. First, the testing method and evaluation method used in Examples and Comparative examples are described below.

"Evaluation (1): Perceived mirror gloss"

The nail enamel was applied on the nails of ten panelists, and after ten minutes sensory evaluation of the perceived mirror gloss was performed by each panelist. The evaluation criteria are as follows:

◎ : 8 or more panelists perceived a mirror gloss.
○ : 5 or more and less than 8 panelists perceived a mirror gloss.
Δ : 3 or more and less than 5 panelists perceived a mirror gloss.
× : Less than 3 panelists perceived a mirror gloss.

"Evaluation (2): Wear resistance"

The sample was applied on a 1 mm-thick glass plate with a brush and thoroughly dried at ordinary temperatures; a YSS tester (from Yasuda Seiki Seisakusho, Ltd.) was used with a load of 250 gf/cm$^2$ between artificial leather and the prepared coating film; the artificial leather was made to travel on the coating film ten times back and forth, after which changes in the state was visually observed. For measuring the degree of gloss of the coating film applied on the base coat, first a 0.1 mm-thick applicator was used to apply the base coat and, after thorough drying at ordinary temperatures, the sample was applied on top with a brush, followed by a measurement using the same method. The coating film being tested was evaluated by using the following criteria.

○ : There was no change in the mirror gloss before and after the test.
Δ : There were fine scratches on the surface and the gloss decreased a little.
× : The coating film was scraped off and the mirror gloss disappeared.

"Evaluation (3): Wear resistance"

After the coating film was prepared in the same manner as in Evaluation 2, one drop of water was dripped on a glass plate, which was left alone for 10 minutes at ordinary temperatures. After 10 minutes, the states of the coating film and the water drop on the coating film were visually observed and evaluated by using the following criteria.

◎ : Both right after the water drop was dripped and 10 minutes later, the coating film maintained the mirror gloss and the water drop was completely repelled on the coating film.
○ : Both right after the water drop was dripped and 10 minutes later, the coating film maintained the mirror gloss but the water drop spread a little on the coating film.
Δ : Right after the water drop was dripped, the coating film had a mirror gloss, but after 10 minutes the mirror gloss had decreased. The water drop spread a little compared with how it was right after the water drop was dripped.
× : Right after the water drop was dripped the coating film lost mirror gloss and the water drop spread a little compared with how it was right after the water drop was dripped.

"Evaluation (4): Long lasting coverage in actual use

The sample was applied with a brush on the nails of ten panelists, and after one day of use sensory evaluation of the perceived mirror gloss was performed by each panelist. For the samples applied on the base coat for measurement, first the base coat was applied with a brush and after thorough drying for 5 minutes at ordinary temperatures the sample was applied on top with a brush. The evaluation criteria are as follows:

◎ : 8 or more panelists felt a mirror gloss was maintained.
○ : 5 or more and less than 8 panelists felt a mirror gloss was maintained.
Δ : 3 or more and less than 5 panelists felt a mirror gloss was maintained.
× : Less than 3 panelists felt a mirror gloss was maintained.

"Evaluation (5): Stability"

A 20 ml screw tube was filled up to 80% full with the sample solution, and after leaving it alone for a week at 50°C, changes in the appearance were evaluated and the content was applied with a brush to evaluate the state of the coating film. The evaluation criteria are as follows:

◎ : The content was mostly homogeneous and the formed coating film also maintained a mirror gloss.

○ : Some precipitation was observed, but virtual homogeneity was achieved by shaking and the coating film maintained a mirror gloss.

Δ : Precipitation occurred; the precipitate was mixed to some degree by stirring but the mirror gloss of the coating film decreased.

× : Precipitation occurred; the precipitate could not be re-stirred, and the mirror gloss of the coating film was lost, too.

"Examples 1-4, Comparative examples 1-3"

The nail enamels of Examples 1-4 and Comparative examples 1-3, whose compositions are listed in Table 1, were prepared and the aforementioned evaluation tests were performed. The results are also shown in Table 1.

[Table 1]

| Ingredients | Example | | | | Comparative example | | |
|---|---|---|---|---|---|---|---|
| | 1 | 2 | 3 | 4 | 1 | 2 | 3 |
| Ion-exchanged water | - | 20 | - | - | - | - | 10 |
| Ethanol | 20 | - | - | 83 | 74 | 20 | 80 |
| n-butyl acetate | - | - | 20 | - | - | - | - |
| Ethyl cellulose | - | - | - | - | 1 | - | - |
| Silver powder A 1) | - | - | - | - | 25 | - | - |
| Silver dispersion fluid A 2) | - | - | - | - | - | 80 | - |
| Silver dispersion fluid B 3) | 80 | - | - | 17 | - | - | 10 |
| Silver dispersion fluid C 4) | - | 80 | - | - | - | - | - |
| Silver dispersion fluid D 5) | - | - | 80 | - | - | - | - |
| Perceived mirror gloss | ○ | ○ | ○ | Δ | × | × | × |
| Stability | ◎ | ○ | ○ | ○ | Δ | Δ | ◎ |

1) AY-6080 from Tanaka Kikinzoku (average particle size 200-1000 nm)
2) Ag Nano Metal Ink from ULVAC Materials, Inc. (Toluene dispersion fluid containing 30% silver, average particle size 7 nm)
3) Finesphere series from Nippon Paint (Ethanol dispersion fluid containing 30% silver, average particle size 30 nm, 2% polymer pigment dispersant)
4) Finesphere series from Nippon Paint (Water dispersion fluid containing 30% silver, average particle size 30 nm, 2% polymer pigment dispersant)
5) Finesphere series from Nippon Paint (Ethanol dispersion fluid containing 30% silver, average particle size 30 nm, 2% polymer pigment dispersant, modified by removing ethanol, dissolving the solid in butyl acetate and adjusting the silver content to 30%)

(Preparation method)

After each ingredient was weighed they were stirred with a disper and used for the test.

The results shown in Table 1 indicate, as shown in Examples 1-3, that a mirror gloss can be obtained when the metal concentration in the coating film is 90% or more and the noble metal concentration in the nail enamel is 5% or more. If a polymer pigment dispersant is used for the dispersant, stability can be improved.

It also indicates that the mirror gloss is lost when the particle size of the noble metal colloid is too large as in Comparative example 1 or when the metal particle size is under 10 nm as in Comparative example 2.

Next, after formula B base coat (hereafter referred to as "formula 1") was applied, the noble metal colloidal solution was applied for testing. Table 2 shows the compositions of formulas 1 for Examples 5-8. Table 3 shows evaluation results for the noble metal colloidal solutions of Examples 1-3 applied on top of Examples 5-8.

Composition of formula 1

[Table 2]

| Ingredients | Example | | | |
|---|---|---|---|---|
| | 5 | 6 | 7 | 8 |
| Nitrocellulose (solid content 70%) | 18 | 15 | 15 | - |
| Alkyd resin | - | 10 | - | - |
| Toluenesulfonamide resin | - | - | 10 | - |
| Ethyl acetate | 20 | 20 | 20 | - |
| Ethanol | 5 | 5 | 5 | 2 |
| Butyl acetate | Balance | Balance | Balance | - |
| Ion-exchanged water | - | - | - | Balance |
| Polymer emulsion 1) | - | - | - | 80 |
| Diethylene glycol diethyl ether | - | - | - | 8 |
| Hectorite | - | - | - | 0.3 |
| 1) Acrylic polymer emulsion having a Tg of 40˚C and a solid content of 40% | | | | |

(Preparation method)

Formula 1 was obtained by mixing the aforementioned ingredients according to a conventional preparation method.

Evaluation results for the noble metal colloidal solutions of Examples 1-3 applied on top of Examples 5-8.

[Table 3]

| | Sample used for formula 1 | | | | |
|---|---|---|---|---|---|
| | None | Example 5 | Example 6 | Example 7 | Example 8 |
| Evaluation results for the noble metal colloidal solution of Example 1 applied on top | | | | | |
| Perceived mirror gloss | ○ | ◎ | ◎ | ◎ | ◎ |
| Wear resistance | × | Δ | Δ | ○ | Δ |
| Water resistance | × | Δ | ○ | ○ | Δ |
| Long lasting coverage in actual use | × | Δ | Δ | ○ | Δ |
| Evaluation results for the noble metal colloidal solution of Example 2 applied on top | | | | | |
| Perceived mirror gloss | ○ | ◎ | ◎ | ◎ | ◎ |
| Wear resistance | × | Δ | Δ | Δ | Δ |
| Water resistance | × | Δ | Δ | Δ | Δ |
| Long lasting coverage in actual use | × | Δ | Δ | Δ | Δ |
| Evaluation results for the noble metal colloidal solution of Example 3 applied on top | | | | | |
| Perceived mirror gloss | ○ | Δ | Δ | Δ | Δ |
| Wear resistance | × | Δ | Δ | Δ | Δ |

(continued)

| Evaluation results for the noble metal colloidal solution of Example 3 applied on top | | | | | |
|---|---|---|---|---|---|
| Water resistance | × | Δ | Δ | Δ | Δ |
| Long lasting coverage in actual use | - | - | - | - | - |

The results in Table 3 indicate that the perceived mirror gloss increases and wear resistance and water resistance increase when an ethanol solution of the noble metal colloid of Example 1 is applied after the formulas 1 shown in Example 5-8 are used. When the noble metal colloid dispersion fluid of Example 2 is applied, there is not much effect in terms of an improvement in water resistance and wear resistance but perceived mirror gloss increases, indicating a combined effect. On the other hand, when the butyl acetate solution of the noble metal colloid of Example 3 is applied, perceived mirror gloss decreases, rather than increasing. When formula 1 is used, the dispersion medium for the noble metal colloid is preferably water or alcohol.

Next, acetyl triethyl citrate and diisobutyl adipate were added as plasticizers to formula 1 to verify their effect. Table 4 shows the compositions of formulas 1 for Examples 9-12. Table 5 and Table 6 show evaluation results for the noble metal colloidal solutions of Example 3 applied on top of Example 7-13.

[Table 4]

| Ingredients | Example | | | |
|---|---|---|---|---|
| | 9 | 10 | 11 | 12 |
| Nitrocellulose (solid content 70%) | 15 | 15 | 15 | - |
| Phthalic acid type alkyd resin | 10 | - | - | - |
| Toluenesulfonamide resin | 4 | 10 | 10 | - |
| Acetyl triethyl citrate | 4 | 5 | 12 | |
| Dibutyl adipate | | | | 3 |
| Ethyl acetate | 20 | 20 | 20 | - |
| Ethanol | 5 | 5 | 5 | 2 |
| Butyl acetate | Balance | Balance | Balance | - |
| Ion-exchanged water | - | - | - | Balance |
| Polymer emulsion 1) | - | - | - | 80 |
| Diethylene glycol diethyl ether | - | - | - | 8 |
| Hectorite | - | - | - | 0.3 |
| 1) Acrylic polymer emulsion having a Tg of 40°C and a solid content of 40% | | | | |

(Preparation method)

Formula 1 was obtained by mixing the aforementioned ingredients according to a conventional preparation method.

Evaluation results for the noble metal colloidal solution of Example 3 applied on top of formulas 1 of Examples 6, 7, and 9-11

[Table 5]

| | Sample used for formula 1 | | | | |
|---|---|---|---|---|---|
| | Example 6 | Example 7 | Example 9 | Example 10 | Example 11 |
| Perceived mirror gloss | ◎ | ◎ | ◎ | ◎ | ○ |

(continued)

|  | Sample used for formula 1 | | | | |
| --- | --- | --- | --- | --- | --- |
|  | Example 6 | Example 7 | Example 9 | Example 10 | Example 11 |
| Wear resistance | Δ | ○ | ◎ | ◎ | Δ |
| Water resistance | ○ | ○ | ◎ | ◎ | ◎ |
| Long lasting coverage in actual use | Δ | ○ | ○ | ◎ | Δ |

Evaluation results for the noble metal colloidal solution of Example 3 applied on top of formulas 1 of Examples 8 and 12.

[Table 6]

|  | Sample used for formula 1 | |
| --- | --- | --- |
|  | Example 8 | Example 12 |
| Perceived mirror gloss | ◎ | ◎ |
| Wear resistance | Δ | ○ |
| Water resistance | Δ | ○ |
| Long lasting coverage in actual use | Δ | ○ |

The results of Examples 9 and 10 shown in Table 5 indicate that combined use of a plasticizer with formula 1 improves the coating film formed by the noble metal colloid of Example 3 in terms of wear resistance, water resistance, and long lasting coverage in actual use. On the other hand, as shown in Example 11, when the blend ratio of the plasticizer becomes 60% or more of the coating film ingredient, then, although water resistance is satisfactory, the perceived mirror gloss decreases a little, and wear resistance and long lasting coverage in actual use decrease rather than increasing. As shown in Table 6, the addition of a plasticizer improves wear resistance and water resistance also for water type enamel recipes.

Other Examples of the present invention are shown below.

Example 13 Golden color mirror gloss nail enamel

[Formula A: Noble metal colloid solution]

|  | wt% |
| --- | --- |
| Ethanol | 2.0 |
| Finesphere gold series | 98.0 |

(From Nippon Paint. Gold content 10%, average particle size 20 nm, polymer pigment dispersant 1%)
[Formula B: Base coat]

|  | wt% |
| --- | --- |
| Nitrocellulose HIG1/2 | 13.0 |
| Trimellitic acid type alkyd resin | 10.0 |
| Isopropyl alcohol | 5.0 |
| Acetyl tributyl citrate | 3.5 |
| Ethyl acetate | 15.0 |
| Butanol | 0.5 |
| Organic viscous clay mineral | 1.2 |
| Antioxidant | Appropriate amount |
| Butyl acetate | Balance |

(Preparation method and evaluation)

A nail enamel was obtained by mixing the aforementioned ingredients according to a conventional preparation method. The obtained nail enamel had a golden mirror gloss. Combined use of formula B improved the mirror gloss and long lasting coverage.

Example 15 Silver color mirror gloss nail enamel

[Formula A: Noble metal colloid solution]

|  | wt% |
|---|---|
| Ethanol | 10.0 |
| Finesphere series | 90.0 |

(From Nippon Paint. Silver content 30%, ethanol dispersion fluid. Average colloidal particle size 50 nm, polymer pigment dispersant 2%.)
[Formula B: Base coat]

|  | wt% |
|---|---|
| Nitrocellulose HIG1/2 | 13.0 |
| Sucrose benzoate | 10.0 |
| Isopropyl alcohol | 5.0 |
| Acetyl tributyl citrate | 6.0 |
| Ethyl acetate | 15.0 |
| Butanol | 0.5 |
| Organic viscous clay mineral | 1.0 |
| Antioxidant | Appropriate amount |
| Butyl acetate | Balance |

(Preparation method and evaluation)

A nail enamel was obtained by mixing the aforementioned ingredients according to a conventional preparation method. The obtained nail enamel had a silver mirror gloss and sufficiently long lasting coverage. The combined use of formula B improved the mirror gloss and long lasting coverage.

Example 16 Silver color mirror gloss nail enamel

[Formula A: Noble metal colloid solution]

|  | wt% |
|---|---|
| Ethanol | 5.0 |
| Finesphere series | 95.0 |

(From Nippon Paint. Silver content 30%, ethanol dispersion fluid. Average colloidal particle size 50 nm, polymer pigment dispersant 2%.)
[Formula B: Base coat]

|  | wt% |
|---|---|
| Nitrocellulose HIG1/2 | 13.0 |
| Sucrose benzoate | 7.0 |
| Trimellitic acid type alkyd resin | 5.0 |
| Isopropyl alcohol | 4.0 |
| Acetyl tributyl citrate | 6.0 |
| Ethyl acetate | 20.0 |

(continued)

| | wt% |
|---|---|
| Butanol | 1.0 |
| Antioxidant | Appropriate amount |
| Butyl acetate | Balance |

(Preparation method and evaluation)

A nail enamel was obtained by mixing the aforementioned ingredients according to a conventional preparation method. The obtained nail enamel had a silver mirror gloss and sufficiently long lasting coverage. Combined use of formula 1 improved the mirror gloss and long lasting coverage.

INDUSTRIAL APPLICABILITY

The present invention can provide a nail enamel that after application gives a finish having a metallic gloss similar to a mirror surface. Also, a nail enamel having significantly improved durability (water resistance and wear resistance) can be obtained by additionally using a base coat. Also, the mirror gloss will improve and a mirror gloss showing less color tone change over time can be obtained.

**Claims**

1. (a) A nail enamel characteristically containing noble metal colloidal particles having an average particle size of 10-100 nm whose concentration is 5-50 wt% of the total amount of the nail enamel.

2. The nail enamel of claim 1 wherein said noble metal colloidal particles are obtained by using a manufacturing method including a manufacturing process in which a metal compound is reduced in the presence of a polymer pigment dispersion agent to obtain a noble metal colloidal particle solution and a concentration process in which the noble metal colloidal particle solution obtained in said manufacturing process is ultrafiltrated or the solvent is evaporated.

3. A 2-formula type nail enamel comprising the following formula A and formula B.

   Formula A: The nail enamel of claim 1 or 2
   Formula B: A base coat containing (b) a film forming formula and (c) water and/or an organic solvent

4. The 2-formula type nail enamel of claim 3 wherein said formula A additionally contains (d) ethanol.

5. The 2-formula type nail enamel of claim 3 or 4 wherein said formula B contains (e) a plasticizer having an IOB value of 0.3-1.0 and a molecular weight of 500 or less.

6. The 2-formula type nail 1 enamel 1 of claim 3, 4, or 5 wherein (b) film forming agent is a combination of (b1) nitrocellulose and (b2) one, two or more chosen from a group consisting of sucrose benzoate, trimellitic acid type alkyd resin, and toluenesulfon amide resin.

7. The nail enamel of claim 1, 2, 3, 4, 5, or 6 wherein said formula A's noble metal is silver.

8. A manicure method having a step in which said formula B is applied on the nails, followed by a step in which said formula A is applied on the nails.

# EP 1 792 598 A1

<table>
<tr><td colspan="2" align="center">**INTERNATIONAL SEARCH REPORT**</td><td>International application No.<br>PCT/JP2005/017148</td></tr>
</table>

A. CLASSIFICATION OF SUBJECT MATTER
*A61K8/19*(2006.01), *A61Q3/02*(2006.01)

According to International Patent Classification (IPC) or to both national classification and IPC

B. FIELDS SEARCHED

Minimum documentation searched (classification system followed by classification symbols)
*A61K8/19*(2006.01), *A61Q3/02*(2006.01)

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)
CAplus(STN), JOIS

C. DOCUMENTS CONSIDERED TO BE RELEVANT

| Category* | Citation of document, with indication, where appropriate, of the relevant passages | Relevant to claim No. |
|---|---|---|
| X<br>Y | JP 2004-162169 A  (Mitsubishi Materials Corp.),<br>10 June, 2004 (10.06.04),<br>Claims 12, 13; Par. Nos. [0035], [0037]<br>(Family: none) | 1,7<br>2-6,8 |
| Y | JP 2003-103158 A  (Nippon Paint Co., Ltd.),<br>08 April, 2003 (08.04.03),<br>Claims 4 to 7; Par. Nos. [0010], [0023],<br>[0048], [0050]<br>(Family: none) | 1-8 |
| Y | JP 2003-277236 A  (National Institute of<br>Advanced Industrial Science and Technology),<br>02 October, 2003 (02.10.03),<br>Claims 1 to 5; Par. Nos. [0002], [0032];<br>example 1<br>(Family: none) | 1-8 |

[X] Further documents are listed in the continuation of Box C.        [ ] See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>28 October, 2005 (28.10.05) | Date of mailing of the international search report<br>08 November, 2005 (08.11.05) |
|---|---|
| Name and mailing address of the ISA/<br>Japanese Patent Office | Authorized officer |
| Facsimile No. | Telephone No. |

Form PCT/ISA/210 (second sheet) (April 2005)

13

## INTERNATIONAL SEARCH REPORT

| International application No. |
|---|
| PCT/JP2005/017148 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 3-279320 A  (Shiseido Co., Ltd.),<br>10 December, 1991 (10.12.91),<br>Page 1, right column, lines 11 to 12<br>(Family: none) | 3-6,8 |
| Y | JP 5-213719 A  (Shiseido Co., Ltd.),<br>24 August, 1993 (24.08.93),<br>Example 6<br>(Family: none) | 3-6,8 |
| Y | WO 2002/43676 A  (Shiseido Co., Ltd.),<br>06 June, 2002 (06.06.02),<br>Examples 57, 58<br>& CN 1396818 A           & EP 1269970 A1<br>& US 2004/081630 A1 | 3-6,8 |
| A | JP 2004-269536 A  (L'Oreal),<br>30 September, 2004 (30.09.04),<br>& BR 400326 A           & EP 1462085 A1<br>& FR 2852236 A1          & US 2004/241423 A1 | 1-8 |

Form PCT/ISA/210 (continuation of second sheet) (April 2005)

| **INTERNATIONAL SEARCH REPORT** | International application No. |
|---|---|
| | PCT/JP2005/017148 |

```
Claim 7
   Claim 7 contains the wording "A nail enamel set forth in claim 1,
2 ···· wherein the noble metal of the agent A ····", but claim 1 or 2
does not contain the term "agent A".  Thus, the invention of claim 7 is
unclear and does not satisfy the requirement of clearness as provided
for in PCT Article 6.

Claim 8
   Claim 8 contains the terms "the above agent B" and "the above agent
A", but it is unclear what the terms refer to.  Thus, the invention of
claim 8 does not satisfy the requirement of clearness as provided for
in PCT Article 6.
```

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H489416 A
- JP H4173724 A
- JP H1215865 A
- JP 2003103158 A
- JP H5177123 A
- JP 54037082 A
- JP H724746 B

**Non-patent literature cited in the description**

- *Kagaku no Ryoiki,* 1957, vol. 11 (10), 719-725